# EUROPÄISCHE PATENTSCHRIFT

(11) **EP 1 810 228 B1**
(45) Veröffentlichungstag und Bekanntmachung des Hinweises auf die Patenterteilung: **03.08.2016**
(21) Anmeldenummer: 05803452.1
(22) Anmeldetag: 08.11.2005
(51) Int. Cl.: G06M 1/16, G06M 1/22, G06M 1/04, A61M 15/00, B65D 83/14, G06M 1/08, G06M 1/24

(54) **SCHRITTSCHALTWERK**
STEP-BY-STEP MECHANISM
MECANISME PAS A PAS

(30) Priorität: 10.11.2004 DE 102004054179; 18.07.2005 DE 102005033398
(43) Veröffentlichungstag der Anmeldung: 25.07.2007
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim
(86) Internationale Anmeldenummer: PCT/EP2005/055823
(87) Internationale Veröffentlichungsnummer: WO 2006/051073

(56) Entgegenhaltungen:
- EP-A- 0 480 488
- EP-A- 1 065 477
- WO-A-01/28887
- US-A- 5 228 586
- US-A- 5 971 140
- US-A1- 2003 178 020
- US-A1- 2004 211 420

## Beschreibung

Die Erfindung betrifft ein Schrittschaltwerk gemäß Gattungsbegriff des Hauptanspruches.

Solche Schrittschaltwerke, z.B. gestaltet gemäß GB-PS 1317315, finden insbesondere Anwendung in der medizinischen Aerosol-Therapie zur Behandlung von Erkrankungen der Atemwege. Eine in dem Gerät unter Druck stehende Kartusche beinhaltet das zu inhalierende Medikament. Zum Ausstoß desselben ist eine axiale Verschiebung der Kartusche in dem Gerät erforderlich. Das in der Regel die Kartusche nahezu vollständig umfassende Gerät besitzt weiter in üblicher Weise ein Mundstück und / oder einen Adapter zur Inhalation durch die Nase. Es besteht ein Bedürfnis, dem Benutzer eine Zählvorrichtung anzubieten, mittels welcher die verbrauchte oder die in der Kartusche noch vorhandene Medikamentenmenge angezeigt wird. Da bei jeder Kartuschenbetätigung eine definierte Medikamentenmenge abgegeben wird, ist es bekannt, die Zählvorrichtung an die Axialverlagerung der Kartusche in dem Inhaler-Gehäuse zur Medikamentenabgabe zu koppeln. Die dazu bekannte gattungsgemäße Lösung ist zu groß, um sie in den Normal-Geräten unterzubringen. Sie ist im Übrigen schwierig montierbar, insbesondere hinsichtlich der auf Querbolzen zum Schrittschaltwerk-Gehäuse gelagerten und durch getrennte Federn zu belastenden Schrittschaltfinger. Die erreichbare Zählsicherheit reicht für den Medikamenteneinsatz nicht aus. Beim Einsetzen oder Entnehmen, z.B. zu Reinigungszwecken können leicht Fehlzählungen auftreten derart, dass das Schrittzählwerk einen Schritt zählt, der aber tatsächlich kein Ausgabeschritt im Inhaler war.

Auch andererorts besteht ein Bedürfnis an einfach aufgebauten zählwerken, die eigenständig arbeiten.

Aus der EP 480488 ist ein Inhaliergerät mit oberer, in Richtung einer Kartusche verschiebbaren Kappe bekannt, welche Kappe ein Ringteil umfasst, von dem entgegengesetzt gerichtet federnde Schrittschaltfinger schräg ausgehen, deren Enden in Zahnkränze eintreten, wenn man die Kappe in Richtung der Kartusche drückt, so dass die lineare Verlagerung der Kappe in eine Drehbewegung eines Skalenringes umgesetzt ist. Durch an der Kappe vorgesehenes Fenster ist die Verdrehung eines Skalenringes sichtbar. Die Lösung ist aufwendig und insbesondere säuberungstechnisch sehr nachteilig.

Im Hinblick auf den zuvor beschriebenen Stand der Technik wird eine technische Problematik der Erfindung darin gesehen, ein Schrittschaltwerk der in Rede stehenden Art in räumlich günstiger Weise bei vereinfachtem Aufbau hand-habungstechnisch sicherer auszugestalten.

Dies ist zunächst und im Wesentlichen gelöst durch den Gegenstand des An spruches 1, wobei auf ein ringformiges Gehäuse mit Schaltgliedern abgestellt ist, die um in Längsrichtung des Gehäuses liegende Achsen umlaufen. Das Schrittschaltwerk passt vor das untere Ende entsprechender (genormter) Kartuschen eines medikamentösen Inhalers und kann dessen Auslassröhrchen umfassen. Die gewählte Konstruktion benötigt geringsten Platzbedarf und ist auch hinsichtlich der Reinigungsmöglichkeiten und darüber hinaus hinsichtlich der Handhabbarkeit beim Reinigen in vorteilhafter Weise ausgebildet. Außerhalb des Inhalers oder dergleichen ist praktisch ein Zählen nicht möglich. Die Funktion ist nur durch rein mechanisch zusammenwirkende Bauteile gewährleistet, welche zudem weiter bevorzugt aus einem einheitlichen Kunststoffmaterial bestehen können. Dies trägt weiter zu einer vereinfachten Herstellbarkeit, insbesondere bei der Montage, bei. Es ist völlig eigenständig, das heißt, es braucht nicht von anderen Teilen, wie einer Kartusche, mitgeschleppt zu werden. Diese Schaltfinger vollführen auch die Rückstellung nach Betätigung.

Die Gegenstände der weiteren Ansprüche sind in Bezug zu dem Gegenstand des Hauptanspruches und geben vorteilhafte Weiterbildungen an.

So ist weiter vorgesehen, dass das Schrittschaltwerk einen konzentrisch umlaufenden Ring mit außenseitiger Skala aufweist, der über ein Planetenrad-Getriebe von einem ebenfalls konzentrisch umlaufenden, vom Betätigungshub angetriebenen Zahnkranz schrittweise gedreht wird. Die Drehbewegung des Skalenrings ist abgezweigt aus einer Relativverlagerung des Schrittschaltwerkes. Dessen Schaltfinger arbeitet auf einer Bahn rund um das Zentrum. Es braucht lediglich ein Stützabschnitt für die Schaltfingernabe vorgesehen zu sein. Bei einer möglichen Entnahme des Schrittschaltwerkes aus einem Gerät ist wegen des dann fehlenden Stützabschnittes für den Schaltfinger eine versehentliche Betätigung des Schrittschaltwerkes nicht möglich. Die Schrittschaltwerke können als Schüttgut verpackt und verkauft werden. Weiter ist vorgesehen, dass das Planetenrad in einer Bohrung des Skalenrings lagert und das zugehörige Sonnenrad auf einer unterseitig gezahnten Scheibe sitzt. Diese Scheibe steht in Eingriff mit dem Schaltfinger. Weiter greift in die Zahnung ein Rastfinger ein, zur Sicherung der jeweils erreichten Scheiben-Drehstellung. Sonnenrad und gezahnte Scheibe sind bevorzugt einteilig ausgeformt, dies bei koaxialer Ausrichtung. Das Planetenrad-Getriebe gibt bevorzugt den Drehwinkel untersetzt an den Skalenring weiter. Entsprechend sind auf dem Skalenring 200 oder 300 Hubstöße anzeigbar. Weiter ist bevorzugt, dass die auf der äußeren Mantelfläche des Skalenrings angeordnete und vor einem Sichtfenster des Gehäuses laufende Skaleneinteilung des Skalenrings jeweils mehreren Einzel-Drehschritten des Planetenrades entspricht. Diesbezüglich erweist es sich weiterhin als vorteilhaft, dass hinsichtlich der Untersetzung ein Einzel-Drehschritt des Planetenrades nach Durchlauf mehrerer Einzel-Drehschritte des Sonnenrades erfolgt. Das Planetenrad steht weiter radial außen mit einem Zahnkranz in Eingriff. Dies fesselt die jeweilige Drehstellung des Skalenringes. Es ist ein von der unteren Randkante schräg aufwärts gerichtet ausgehenden Schlitz zum Eintritt des Schrittschaltfingers vorhanden, um hierüber die Schaltrichtung des Fingers zum schrittweisen Weiterdrehen der sonnenradseitigen Scheibe vorzugeben. Der oder die Schrittschaltfinger Bestandteil eines Schrittschaltfinger-Sterns, welcher. eine zentrale Nabe aufweist, an der radial hierzu beabstandet diametral gegenüberliegende Schrittschaltfinger angeformt sind. Dieser Schrittschaltfinger-Stern ist bevorzugt einstückig geformt, bevorzugt aus einem Kunststoffmaterial. Die Schrittschaltfinger sind schräg aufwärts von der Nabe ausgehend gerichtet, wobei weiter die Schrittschaltfinger sich beim Betätigungshub in Richtung einer Ebene senkrecht zur Längsachse bewegen. Demzufolge werden die Schrittschaltfinger im Zuge der Ausgabebewegung, d.h. im Zuge der Verlagerung, z.B. einer Kartusche entlang ihrer Längsmittelachse, gespannt. Es bewegt sich hierbei das gesamte SchrittschaltwerkGehäuse. Die Nabe des Schrittschaltfinger-Sterns kann beim Einsatz in normalen Inhalern auf der Stirnfläche des handgerätgehäuseseitigen Stützabschnitts aufsitzen. Dieser Stützabschnitt besitzt dann eine Doppelfunktion: zum einen als Auslöseelement in Verbindung mit dem Ventilrohr der Kartusche, zum weiteren hat der Stützabschnitt die Funktion eines Widerlagers für den Schrittschaltfinger-Stern. Alles mit der Konsequenz, dass man das Schrittschaltwerk außerhalb eines Gerätes gar nicht betätigen kann. Das Schrittschaltwerkgehäuse mit den weiteren Schaltgliedern wird relativ zu dem Schrittschaltfinger-Stern verlagert, dies unter Bewegung der Schrittschaltfinger in die senkrecht zur Längsachse ausgerichteten Ebene, was die beschriebene Vorspannung der Schrittschaltfinger und das Weiterschalten des Zählwerkes bewirkt. Nach einem Loslassen des Betätigungsdruckes stellen sich die Finger wieder zurück in ihre Grundstellung. Dies ist durch die vorgespannten Schrittschaltfinger erreicht, die ihre ursprüngliche, schräg aufwärts gerichtete Position selbsttätig wieder einnehmen und hierbei die Schaltglieder sowie das Schrittschaltwerkgehäuse in ihre Ursprungslage drücken. Entsprechend ist das Schrittschaltwerk hinsichtlich der Rückverlagerung in eine Grundstellung entkoppelt von der Kartusche. Es verfügt über eine durch die Schrittschaltfinger gebildete Rückstellfeder. Bevorzugt ist das gesamte Schrittschaltwerk nebst Skalenring in einem runden Gehäuse gefasst, was wiederum hinsichtlich der gewünschten Reinigung des Gehäuses sich als Vorteil erweist Es liegen keine etwaige beim Reinigen bspw. durch Bürsten zu beschädigende Getriebeteile frei. Vielmehr ist eine kompakte, im Wesentlichen geschlossene Bauform gewählt. Diesbezüglich wird weiter vorgeschlagen, dass das als Flachteller gestaltete Schrittschaltwerkgehäuse ein zentrales Loch zum Durchtritt eines Bauteiles besitzt. Das Schrittschaltwerkgehäuse kann zusätzlich auch an der Kartusche zentral unterhalb der öffnungsseitigen Stirnwand in Überlappung zum Kartuschen-Ventilrohr-wiebekannt-festlegbar sein durch federnde Verrastung.

Nachstehend ist die Erfindung anhand der beigefügten Zeichnung, welche lediglich verschiedene Ausführungsbeispiele darstellt, näher erläutert. Es zeigt:
- Fig.1: in perspektivischer Explosionsdarstellung das Schrittschaltwerk für das erfindungsgemäße Schrittschaltwerk;
- Fig. 2: einen Querschnitt durch das Schrittschaltwerk;
- Fig. 3: in einer Längsschnittdarstellung ein Inhaler-Handgerät mit einem an dessen Kartusche verrasteten, schematisch dargestellten Schrittschaltwerk;
- Fig. 4: eine der Fig. 3 entsprechende Schnittdarstellung, jedoch eine andere Lösung betreffend, bei welcher das schematisch dargestellte Schrittschaltwerk an einem Gehäuse verrastet ist;
- Fig. 4a: eine abgewandelte Ausführungsform in einer Schnittdarstellung gemäß Fig. 4, bei welcher das Schrittschaltwerk in einem Inhaler gegen Abziehen blockiert ist;
- Fig. 5: eine Längsschnittdarstellung eines Inhalers mit eingesetztem Schrittschaltwerk, nach Trennen eines oberen Inhaler-Gehäuseteils von seinem Mundstück.

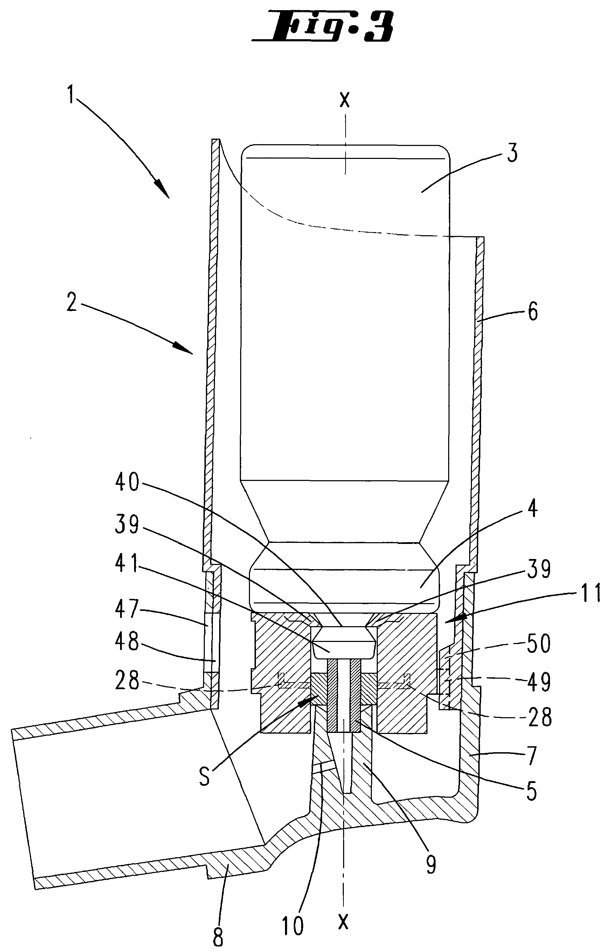

Das in Fig. 3 in einer schematischen Schnittdarstellung gezeigte Handgerät 1 dient zur portionierten Ausgabe sprühfähiger Substanzen, insbesondere von Inhaliermedikamenten.

Hierzu weist das Handgerät 1 zunächst ein Handgerätgehäuse 2 auf, in welches eine die sprühfähige Substanz beinhaltende Kartusche 3 einsetzbar ist. Diese Kartusche 3 ist in dem Gehäuse 2 axial verschiebbar. In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Gehäuse 2 erreicht. Das Inhaler-Gehäuse 2 ist zweigeteilt und besteht aus zwei übereinander angeordneten Ringteilen 6 und 7, von welchen das obere Ringteil 6 schaftartig ausgeformt ist und das untere Ringteil 7 ein etwa quer zur Schafterstreckung ausgerichtetes Mundstück 8 aufweist. Letzteres ist durch eine nicht dargestellte Abdeckkappe verschließbar. Das Ventilrohr 5 der Kartusche 3 stützt sich in einem zugeordneten rohrförmigen Stützabschnitt 9 innerhalb des unteren Ringteiles 7 ab, dies bei axialer Beweglichkeit der Kartusche 3 innerhalb des die Kartusche 3 umgebenden, schaft-artigen Ringteils 6. Der das Ventilrohr 5 der Kartusche 3 klemmend aufnehmende, innerhalb des unteren Gehäuse-Ringteils 7 ausgeformte Stützabschnitt 9 ist mit einem gegenüber einen das Ventilrohrende aufnehmenden Abschnitt durchmesserverringerten Strömungskanal 10 versehen, welcher strömungstechnisch in Verbindung steht mit dem Ventilrohr 5, wobei das dem Ventilrohr 5 abgewandte Ende des Strömungskanals 10 in Richtung auf das Mundstück 8 weist.

Zentral unterhalb der öffnungsseitigen Stirnwand der Kartusche 3 ist in Überlappung zum Kartuschen-Ventilrohr 5 ein Schrittschaltwerk 11 angeordnet. Dieses dient zum Registrieren und / oder Zählen / Anzeigen der durchgeführten Ausgabebetätigungen, dies in Abhängigkeit von den durchgeführten Öffnungshüben der Kartusche 3.

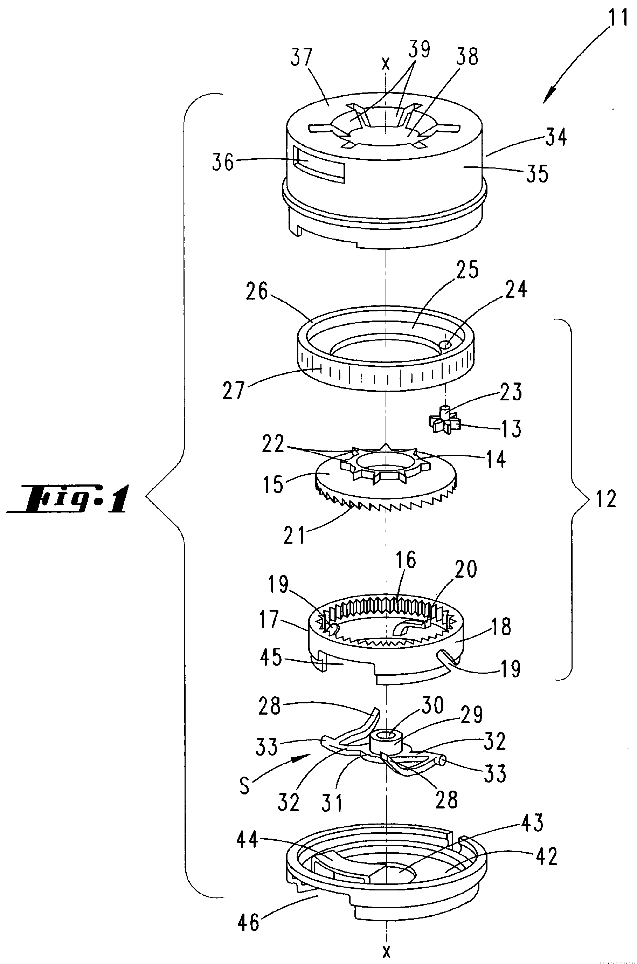

Das Schrittschaltwerk 11 ist in Fig.1 in einer perspektivischen Explosionsdarstellung gezeigt. Zentraler Bestandteil des Schrittschaltwerks 11 ist ein Planetenrad-Getriebe 12, bestehend aus einem Planetenrad 13, einem Sonnenrad 14, welches auf einer unterseitig gezahnten Scheibe 15 sitzt und einem mit dem Planetenrad 13 fesselnd zusammenwirkenden Zahnkranz 16. Letzterer ist wandungsinnenseitig eines nicht drehbar gehalterten, rohrabschnittförmigen Ringes 17 ausgeformt. Die Mantelwandung 18 des Ringes 17 ist in diametral gegenüberliegenden Bereichen durchsetzt von schräg aufwärts in Schaltrichtung gerichtet ausgehenden Schlitzen 19, die nach unten zur dem Zahnkranz 16 abgewandten Ringkante hin offen auslaufen.

Der Zahnkranz 16 erstreckt sich in axialer Richtung etwa über die halbe Höhe des Ringes 17, dessen Mantelwandung 18 zur dem Zahnkranz 16 abgewandten Ringstirnkante hin abgestuft, sich radial verjüngend ausgebildet ist.

Unterhalb des Zahnkranzes 16 ist innenseitig der Mantelwandung 18 des Ringes 17 ein Rastfinger 20 angeformt. Dieser ist mit Bezug auf einen Grundriss des Ringes gegenüber dem Zahnkranz 16 nach radial innen versetzt; greift entsprechend in einen zum Zahnkranz 16 nach radial innen eingezogenen Kreisraum. Weiter ist die Anordnung des in etwa in Vertikalrichtung elastisch ausgebildeten Rastfingers 20 so gewählt, dass dieser etwa in eine durch die unteren Randkanten des Zahnkranzes 16 aufgespannte Horizontalebene eingreift.

Der Durchmesser der das Sonnenrad 14 tragenden Scheibe 15 ist geringfügig kleiner gewählt, als der Innendurchmesser des Ringes 17 im Bereich des Zahnkranzes 16. Sonnenrad 14 und Scheibe 15 sind bevorzugt einstückig, materialeinheitlich ausgebildet.

Unterseitig der Scheibe 15 ist eine randbezogen umlaufende Sägezahnung 21 vorgesehen, in welche der Rastfinger 20 des Ringes 17 als Halteglied (gegen Rückdrehen) eingreift.

Das Sonnenrad 14 weist eine grobe Verzahnung auf. So sind in dem dargestellten Ausführungsbeispiel über den Umfang des Sonnenrades 14 acht Sonnenrad-Zähne 22 gleichmäßig verteilt ausgeformt. Diese Zähne 22 wirken im Zuge der Sonnenrad-Umdrehung mit dem in gleicher Ebene zwischen dem Sonnenrad 14 und dem Zahnkranz 16 des Ringes 17 angeordneten Planetenrad 13 zusammen.

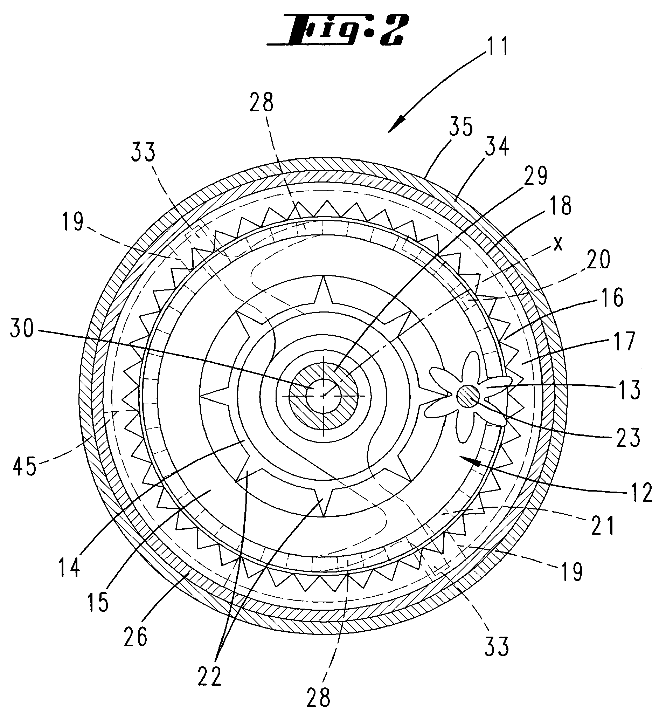

Das Planetenrad 13 besitzt einen einseitig nach oben, d. h. von der Scheibe 15 des Sonnenrades 14 weg abragenden Achszapfen 23. Dieser ist in einer Bohrung 24 im Bereich eines scheibenartig nach radial innen weisenden Kragens 25 eines Skalenringes 26 drehbar gefasst. Der Skalenring 26 ist mantelaußenwandig mit einer umlaufenden Skaleneinteilung 27 versehen, wobei die Skaleneinteilung jeweils mehreren Einzel-Drehschritten des den Skalenring 26 weiterführenden Planetenrades 13 entspricht. Das Planetenrad kämmt zusätzlich mit dem Zahnkranz 16 (siehe Fig. 2), um Zwischenstellungen des Skalenringes zu sichern.

Die schrittweise Verlagerung des Sonnenrades 14 bzw. der einstückig hiermit ausgeformten Scheibe 15 erfolgt über etwa vertikal federnd ausweichbar ausgebildete Schrittschaltfinger 28. Diese greifen unterseitig in die Sägezahnung 21 der Scheibe 15.

Die Schrittschaltfinger 28 sind mit Bezug zu der Hauptachse x des gesamten Schrittschaltwerkes 11 diametral gegenüberliegend angeordnet. Hierzu ist zunächst ein zylinderförmiger Zentralkörper in Form einer Nabe 29 vorgesehen mit einer zentralen axialen Durchgangsbohrung 30. Deren Durchmesser ist geringfügig größer bemessen als der Außendurchmesser des diese Durchgangsbohrung 30 zu durchsetzenden Kartuschen-Ventilrohres 5.

Fußseitig geht die Nabe 29 über in einen radial erweiterten Kragen 31. An diesem sind diametral gegenüberliegend in Radialrichtung abragende Führungsabschnitte 32 angeformt, die jeweils im Bereich ihrer freien Enden einen in dem zugeordneten Schlitz 19 des Ringes 17 einliegenden Führungszapfen 33 ausformen. Nabe und Finger 28 liegen im Innenraum des Ringes 17 und gegenüber der Bodenfläche des Gehäuses 34 rückversetzt.

Die Schrittschaltfinger 28 wurzeln jeweils mit einem Horizontalabschnitt an den Führungsabschnitten 32 unter Belassen der über den horizontalen Abschnitt radial außen abragenden Führungszapfen 33. Die von den Horizontalabschnitten abragenden Schrittschaltfinger 28 gehen schräg aufwärts gerichtet aus, etwa unter Einschließen eines Winkels von 45 Grad zur Horizontalen, angepasst an die Schrägung der Schlitze 19 im Ring 17. Der so gebildete Schrittschaltfinger-Stern trägt das Bezugszeichen S.

Der Schrittschaltfinger-Stern S, der den inneren Zahnkranz 16 aufweisende Ring 17, die einstückig mit dem Sonnenrad 14 ausgeformte Scheibe 15 sowie der Skalenring 26 sind konzentrisch zueinander auf der Achse x ausgerichtet, wobei die Höhe des Ringes 17 so gewählt ist, dass sowohl der Schrittschaltfinger-Stern S als auch das Sonnenrad 14 samt Scheibe 15 in diesem aufgenommen sind.

Das gesamte Planeten-Getriebe 12, sowie der Schrittschaltfinger-Stern S und der Skalenring 26 sind aufgenommen in einem topfartigen Schrittschaltwerkgehäuse 34 mit einem Außendurchmesser, welcher an den Außendurchmesser der Kartusche 3 angepasst ist.

Das Gehäuse 34 besitzt eine Mantelwandung 35. Diese weist ein Sichtfenster 36 auf, durch welches die Skaleneinteilung 27 des Skalenrings 26 erkennbar ist.

Die Gehäusedecke 37 besitzt eine zentrale Durchbrechung 38, welche in der in den Figuren 1 bis 3 gezeigten Ausführungsform umfasst ist von konisch zum Gehäuseinnern hin zulaufenden Rast-Federzungen in der in den Figuren 1 bis 3 gezeigten Ausführungsform 39. Der Durchbrechungs-Durchmesser ist angepasst an einen Durchmesser eines Taillenabschnittes 40 eines über die öffnungsseitige Stirnwand der Kartusche 3 zentral überragenden Kragens 41, aus welch letzterem das Ventilrohr 5 auswächst.

Der Gehäuseboden 42 ist gebildet durch ein gesondertes Teil. Dieses ist unter Aufnahme der vorbeschriebenen Schrittschaltwerk-Einzelteile mit dem Gehäuse 34 verbunden, so bspw. mit diesem verschweißt, verklipst oder über eine Presspassung an diesem gehaltert.

Der tellerartige Gehäuseboden 42 besitzt eine zentrale Bohrung zum Durchtritt eines Domes z.B. des Ventilrohres 5. Des Weiteren ist auf dem Gehäuseboden 42 ein Raststück 44 ausgeformt, welches zur lageorientierten Fesselung des Ringes 17 in eine entsprechend in dessen Mantelwandung 18 ausgeformte, fensterartige Ausnehmung 45 greift.

Im selben Winkelbereich, in welchem das Passstück 44 auf dem Boden angeordnet ist, weist die Mantelaußenwandung des Gehäusebodens 42 einen Freischnitt 46 auf. Diese ist im Einbauzustand dem Bereich des Ausgangsquerschnittes des Strömungskanals 10 im unteren Ringteilgehäuse 7 zugeordnet.

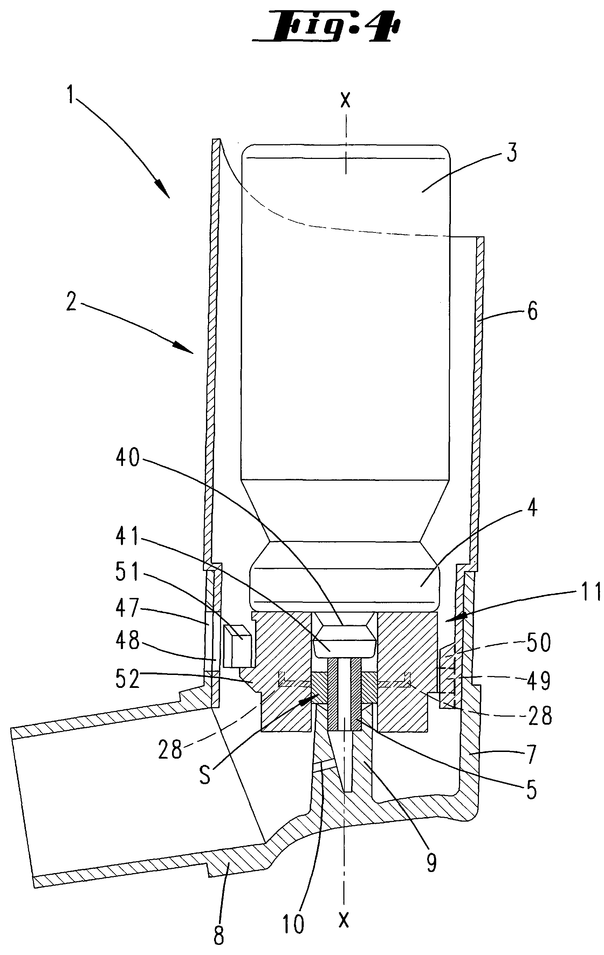

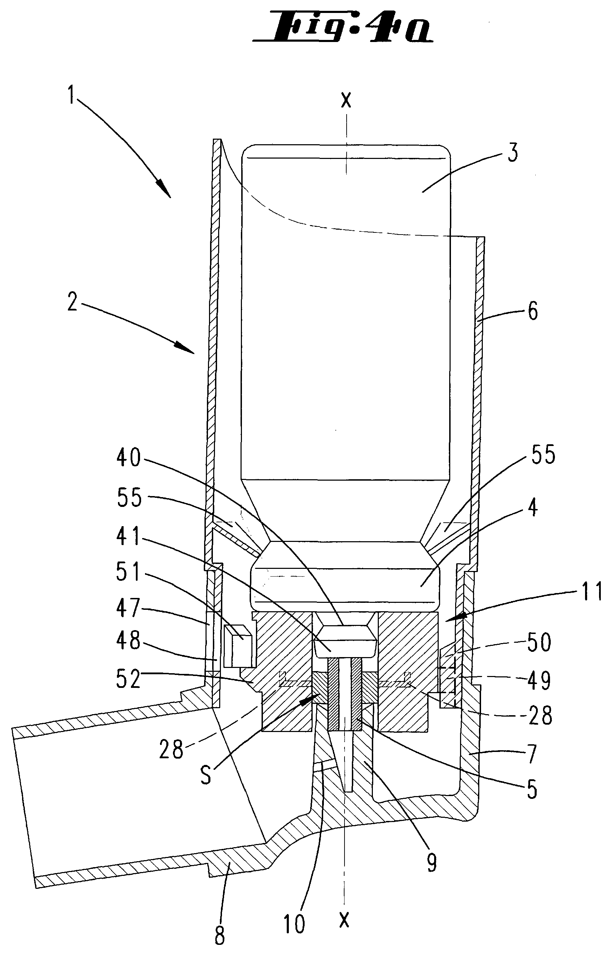

Die Funktionsweise des Schrittschaltwerkes 11 ist unabhängig von der anhand der Figuren 3 und 4 noch zu beschreibenden Anordnung grundsätzlich wie folgt:
Die Schaltglieder (Schrittschaltfinger-Stern S, Ring 17, Scheibe 15, Planetenrad 13 und Skalenring 26) wie auch das Gehäuse 34 mit dem Gehäuseboden 42 sind auf Achsen angeordnet, die sich in Längsrichtung des Gehäuses 34, also des Betätigungshubes x - x, im Beispiel auch der Kartusche 3, erstrecken. Mit Ausnahme des Planetenrades 13 sind sogar alle weiteren Bauteile des Schrittschaltwerkes 11 auf der Kartuschenlängsachse x - x positioniert.

Das Schrittschaltwerk 11 lässt sich einfachst entsprechend konzentrisch im Schatten eines Bauteiles, z.B. der Kartusche 3 anordnen, dies konkret in dem zwischen Kartuschenkopf 4 und Stützabschnitt 9 eines genormten Inhalers 2 belassenen Bauraum. Das Schrittschaltwerk 11 stützt sich mit der in dem Schaltwerkgehäuse 34 zentral gelagerten Nabe 29 des Schrittschaltfinger-Sterns S auf der Stirnfläche des Stützabschnitts 9 des Inhaliergehäuses 2 ab. Das die Nabe 29 durchsetzende Ventilrohr 5 bietet eine zusätzliche Zentrierung der gesamten Schrittschaltwerk-Einheit.

Bei Durchführung eines Betätigungshubs der Kartusche 3 und damit einhergehender Vertikalverlagerung des Gehäuses 34 in Richtung auf einen Stützabschnitt 9 wird das Schaltwerkgehäuse 34 über den Kartuschenkopf 4 mitgeschleppt, dies unter Relativverlagerung des Gehäuses 34, des Planetenrad-Getriebes 12 und des Skalenringes 26 zu dem Schrittschaltfinger-Stern S, welcher eine Abstützung auf einem Stützabschnitt, hier Nr. 9, erfährt. Infolgedessen bewirken die sich spannenden Schrittschaltfinger 28 weiter unterstützt durch Drehaufgleiten des Schrittschaltfinger-Sterns S in den mantelwandseitigen Schlitzen 19 des Ringes 17 einen schrittweisen Drehvorschub der sägeverzahnten Scheibe 15. Einhergehend damit dreht sich das Sonnenrad 14 um denselben Winkelbetrag. Die Schrittschaltfinger 28 bewegen sich hierbei aus der schrägen Ausrichtung in Richtung auf eine senkrecht zur Längsachse x - x ausgerichtete Ebene.

Wenn das Sonnenrad 14 z.B. lediglich acht gleichmäßig über den Umfang verteilt angeordnete Zähne aufweist, führt nicht jede Schritt-Drehbewegung des Sonnenrades 14 zwangsläufig zu einer Drehbewegung des Planetenrades 13. Vielmehr wird die Drehung des Planetenrades 13 um dessen Achse und eine die damit einhergehende Drehverlagerung des Skalenringes 26 erst nach mehreren Einzel-Drehschritten des Sonnenrades 14 durchgeführt. Diese Summierungsfunktion kann vorteilhafter sein, als die 1:1-Übersetzung.

Gemäß der Darstellung in Fig. 3 ist in bestimmten Fällen das gesamte Schrittschaltwerk 11 mittels des Gehäuses 34 leicht an einem Kragen 41 rastend festlegbar. Zugeordnet zu dem gehäuseseitigen Sichtfenster 36 eines Inhalers weisen die zugeordneten Abschnitte der Gehäuseringteile 6 und 7 dann gleichfalls Sichtfenster 47,48 auf, welche durch die gewählte Position, zugewandt dem Mundstück 8 des Gehäuses 2, im Blickfeld des das Handgerät 1 bedienenden Benutzers liegen. Zur lageorientierten Befestigung des Schrittschaltwerkes 11 ist dieses mit einem radial von dem Gehäuse 34 abragenden Führungsblatt 49 versehen.

Die Befestigungs-Verrastung des Schrittschaltwerks 11 ist so gewählt, dass bei einer Entnahme der Kartusche 3 aus dem Gehäuse 2 das Schrittschaltwerk 11 an der Kartusche 3 verbleibend mit ausgezogen wird. Dies ist zufolge der besonderen Konstruktion des Schrittschaltwerkes selbst im Arznei-Sektor durchaus möglich, weil das Schrittschaltwerk im entnommenen Zustand praktisch nicht betätigbar ist, sich selbst also nicht verstellen kann.

Alternativ besteht deshalb auch die Möglichkeit, wie in Fig. 4 schematisch dargestellt, dass das Schrittschaltwerk 11 mit dem Gehäuse 2 verrastet ist. Hierzu überfährt das Schrittschaltwerk 11 im Zuge einer Erstbestückung einen oder mehrere radial nach innen einragende Rastvorsprünge 51 des Gehäuses 2, welche Rastvorsprünge 51 hiernach von radial von dem Schaltwerkgehäuse 34 abragenden Kragenabschnitten 52 unterfangen sind. Die Rastvorsprünge 51 sind in ihrer Vertikalausrichtung so positioniert, dass die Vertikalverlagerbarkeit des Schrittschaltwerkes 11 bei Hubbetätigung der Kartusche 3 gewährleistet ist. Durch die gewählte Anordnung formen die Rastvorsprünge 51 Niederhalter aus, welche das Schrittschaltwerk 11 bei einem Abziehen der Kartusche 3 in dem Gehäuse 2 zurückhalten. Es kann sich auch dann nicht verstellen.

In einer weiteren, nicht dargestellten Ausführungsform kann eine Kombination der Ausführungsformen gemäß den Darstellungen in den Figuren 3 und 4 vorgesehen sein, bei welcher das Schrittschaltwerk 11 mittels der Rast-Federzunge 39 an dem kartuschenkopfseitigen Kragen 41 rastfestgelegt ist. Eine solche vormontierte Kartuschen-Schrittschaltwerk-Einheit wird vor einer Erstbenutzung des Inhalers in dessen Gehäuse 2 eingeführt, wobei an dem SchaltwerkGehäuse 34 radial abragende Kragenabschnitte 52 gerätegehäuseseitige Rastvorsprünge 51 entsprechend dem zweiten Ausführungsbeispiel überlaufen, was zu einer endgültigen Festlegung des Schrittschaltwerkes 11 in dem Gehäuse 2 führt. Diese Verrastung zwischen Schrittschaltwerk 11 und Inhaler-Gehäuse 2 ist stärker gewählt als die Verrastung zwischen Schrittschaltwerk 11 und Kartusche 3, demzufolge ein Abziehen der Kartusche 3 nach einer Erstbenutzung zu einer Rastaufhebung zwischen Kartusche 3 und Schrittschaltwerk 11 führt. Das Wiedereinsetzen der Kartusche 3 ist durch eine relativ schwache Rastfederausformung zur Zusammenwirkung mit dem kartuschenkopfseitigen Kragen 41 erleichtert.

Sowohl bei der vorbeschriebenen Ausführungsform als auch bei der Ausführungsform gemäß der Darstellung in Fig. 4 besteht die Möglichkeit, die Verrastung zwischen Gerätegehäuse 2 und Schrittschaltwerk 11 nach Entfernen der Kartusche 3 aus dem Gehäuse 2 für sich aufzulösen.

Die Darstellung in Fig. 4a zeigt eine weitere Ausführungsform, bauend auf der in Fig. 4 gezeigten Ausgestaltung. So ist zur weiteren Festlegung der Kartusche 3 - neben einer üblichen Klemmhalterung des Ventilrohrs 5 in dem handgerätgehäuseseitigen Stützabschnitt 9 - eine Blockierung der Kartusche 3 im Bereich des oberen Ringteil-Gehäuses 6 vorgesehen. So ragen mantelinnenseitig dieses oberen Gehäuseringteiles 6 schräg abwärts gerichtete Rückhaltefinger 55 ab, welche materialeinheitlich, einstückig mit dem oberen Gehäuseteil 6 gebildet sind. Diese Rückhaltefinger 55 sind so positioniert, dass deren freien Randkanten in der Zuordnungsstellung zur Kartusche 3 in den hinter dem Kartuschenkopf 4 ausgeformten Taillenbereich der Kartusche 3 sperrend eintreten, um so die Kartusche 3 zu blockieren. Weiter sind die Rückhaltefinger 55 derart ausgeformt, dass diese zumindest bei einer Erstbestückung des Gehäuses 2 mit der Kartusche 3 durch den Kartuschenkopf 4 überlaufen werden können. Gezählt wird bei diesem Einsetzen aber auch wiederum nicht.

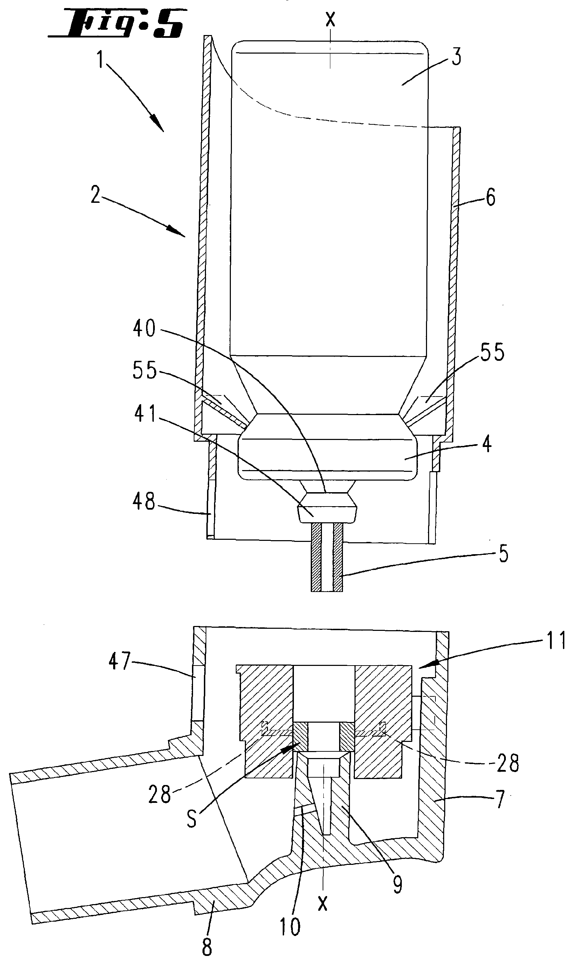

Die Darstellung in Fig. 5 zeigt eine weitere Ausführungsform. In dieser ist auch die Kartusche 3 mittels Rückhaltefingern 55 an dem oberen Inhaler-Gehäuseteil 6 gehaltert. Dieses obere Inhaler-Gehäuseteil 6 ist von dem unteren Gehäuseteil 7, welches das Mundstück 8 ausformt, lösbar, wobei die Trennung der beiden Gehäuseteile 6 und 7 etwa im Bereich der Position des tellerförmigen Schrittschaltwerkgehäuses 34 vorgenommen wird. In der zusammengesetzten Stellung der beiden Gehäuseteile 6 und 7 sind diese bevorzugt verrastet, wozu ein Gehäuseteil eine Rastnase und das andere Gehäuseteil eine entsprechend platzierte Rastaufnahme aufweist.

Zufolge dieser ermöglichten Trennung ist eine verbesserte Reinigung des Mundstückes 8, insbesondere des den Stufenabschnitt 9 aufweisenden Knickabschnittes erreicht. Dies ist weiter noch dadurch erleichtert, dass das gesamte Schrittschaltwerk 11, welches als kompakte Baugruppe geformt ist, in einfachster Weise aus dem unteren Gehäuseteil 7 entnehmbar ist und so einer gesonderten Reinigung zugeführt werden kann, ohne die Gefahr etwaiger Zählschritte.

In dem dargestellten Ausführungsbeispiel sind keine Festlegungsmittel - wie bspw. Rastvorsprünge 51, welche mit Kragenabschnitten 52 zusammenwirken, vorgesehen. Vielmehr ist eine Festlegung des gesamten Schrittschaltwerks 11 in dem unteren Gehäuseteil 7 in Zusammenwirkung mit der Kartusche 3 in der Gebrauchsstellung erreicht, dies unter Ausrichtung des Schrittschaltwerks 11 zwischen dem Stützabschnitt 9 und der zugewandten Stirnfläche des Kartuschenkopfes 4 (wie auch anhand der in Fig. 4a gezeigten Ausführungsform dargestellt). Auch beim Waschen etc. des herausgenommenen Schrittschaltwerkes besteht keinerlei Beschädigungsgefahr.

Auch in dieser Ausführungsform ist das Schrittschaltwerk 11 gegen Drehverlagerung um die Achse x - x durch einen Formschluss zwischen Schrittschaltwerk 11 und unterem Gehäuseteil 7 gesichert.

Alle offenbarten Merkmale sind (für sich) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Schrittschaltwerk, insbesondere für Handgeräte (1) zur portionierten Ausgabe von Inhaliermedikamenten, mit einem ringförmigen Gehäuse, um dessen Längsachse (x-x) ein schrittweise bewegter Skalenring (27) umläuft, veranlasst durch Schaltfinger, die beim Betätigungshub entgegen Federbelastung in Richtung einer Ebene senkrecht zur Längsachse (x-x) des Gehäuses (34) verschwenken und dadurch über Zahnung die Drehung des Skalenringes veranlassen, **dadurch gekennzeichnet, dass** ein drehfest im Gehäuse (34) angeordnetes Ringteil (17) mindestens einen von der unteren Randkante schräg aufwärts ausgehenden Schlitz (19) zum Eintritt eines Führungszapfens (33) eines Schritschaltfinger-Sternes (S) aufweist, dessen von einer zentralen Nabe (29) sekantenförmig entgegengesetzt schräg aufwärts gerichtet ausgehende Schrittschaltfinger (28) sich beim Betätigungshub entgegen ihrer Eigenfederspannung einschwenkend in den Zahnung (21) zur Drehung des Skalenringes (26) treten.

2. Schrittschaltwerk nach Anspruch 1, **dadurch gekennzeichnet, dass** die Drehung des Skalenringes (26) über ein Planetenrad-Getriebe (12) erfolgt, dessen Planetenrad (13) in einer Bohrung (24) des Skalenringes (26) lagert und dessen zugehöriges Sonnenrad (14) auf einer unterseitig gezahnten Scheibe (15) sitzt, in deren Zähne die Schrittschaltfinger (28) eingreifen, wobei das Planetenrad-Getriebe (12) den Drehwinkel der Scheibe (15) untersetzt an den Skalenring (26) weitergibt.

3. Schrittschaltwerk nach einem oder mehreren der vorhergehenden Ansprüche, **dadurch gekennzeichnet, dass** die Nabe (29) des Schrittschaltfinger-Sternes (S) eine zentrale Bohrung besitzt und mit ihrer unterseitigen Stirnfläche einem Stützabschnitt (9) zugeordnet ist.

4. Schrittschaltwerk nach einem oder mehreren der vorhergehenden Ansprüche, **dadurch gekennzeichnet, dass** die Achsen sämtlicher Schaltglieder senkrecht zur Ebene des Skalenringes (26) stehen.

5. Schrittschaltwerk nach einem oder mehreren der vorhergehenden Ansprüche, **dadurch gekennzeichnet, dass** die Nabe (30) mit Abstand von der Unterseite des Bodens des Gehäuses (34) liegt.

6. Schrittschaltwerk nach einem oder mehreren der vorhergehenden Ansprüche, **dadurch gekennzeichnet, dass** der in die Gehäusedecke (37) eingeleitete Betätigungshub sich über den Skalenring, das Planetenrad-Getriebe (12) und über die Zahnung (21) bis in die nahezu flachliegenden Schaltfinger (28) fortsetzt, welche durch Eingriff in die Zahnung (21) der Scheibe (15), letztere unter Überspringen der Rückdrehstop-Finger (20), dreht.

7. Schrittschaltwerk nach einem oder mehreren der vorhergehenden Ansprüche, **gekennzeichnet durch** die Anordnung in einem Handgeräte-Inhaler derart, dass das als Flachteller gestaltete Schrittschaltwerkgehäuse (34) einschließlich der Nabe (29) ein zentrales Loch (38) zum Durchtritt des InhalerVentilrohres (5) besitzt und an dem Handgerätgehäuse (2) derart verrastet ist, dass es noch um den Betätigungshub einwärts verlagerbar ist unter Abstützung der unteren Stirnfläche der Nabe (29) auf dem Stützabschnitt (9), welcher das zentrale Loch (38) aufweist.

8. Handgerät zur portionierten Ausgabe von Inhaliermedikamenten durch das Auslassröhrchen einer dazu entgegen Federbelastung verlagerbaren Kartusche, **gekennzeichnet durch** die Anordnung eines Schrittschaltwerkes gemäß Anspruch 1 gefesselt an die Kartusche und derart, dass die Federbelastung des Kartuschen-Auslassröhrchens gleichgerichtet ist zur Eigenfederspannung der Schrittschaltfinger.

## Claims

1. Step-action indexing mechanism, in particular for hand-held units (1) for apportioned delivery of inhaler medicaments, having an annular housing encircled, around its longitudinal axis (x-x), by a scale ring (27) which is moved in a stepwise manner by indexing fingers which pivot, counter to spring loading, in the direction of a plane perpendicular to the longitudinal axis (x-x) of the housing (34) during the actuating stroke and thus via toothing formation means cause the scale ring to rotate, **characterized in that** an annular part (17), which is disposed in a rotationally fixed manner in the housing (34), has at least one slot (19) which extends obliquely upward from the lower peripheral edge for the insertion of a guide pin (33) of a step-action indexing-finger star (S), the step-action indexing fingers (28) of which extend from a central hub (29), in opposite directions, directed obliquely upward in the form of a secant, and, during the actuating stroke, counter to their inherent spring stressing, enter, moving swiveling inward into the toothing formation (21) in order to rotate the scale ring (26).

2. Step-action indexing mechanism according to Claim 1, **characterized in that** the scale ring (26) is rotated via a planet-gear mechanism (12), the planet gear (13) of which is mounted in a bore (24) of the scale ring (26) and the associated sun gear (14) of which is seated on a disk (15) which is toothed on the underside and in the teeth of which the step-action indexing fingers (28) engage, the planet-gear mechanism (12) passing on the angle of rotation of the disk (15) to the scale ring (26) in stepped-down form.

3. Step-action indexing mechanism according to one or more of the preceding claims, **characterized in that** the hub (29) of the step-action indexing-finger star (S) has a central bore and, by way of its underside end surface, is associated with a supporting portion (9).

4. Step-action indexing mechanism according to one or more of the preceding claims, **characterized in that** the axes of all the indexing members are located perpendicularly to the plane of the scale ring (26).

5. Step-action indexing mechanism according to one or more of the preceding claims, **characterized in that** the hub (30) is spaced apart from the underside of the base of the housing (34).

6. Step-action indexing mechanism according to one or more of the preceding claims, **characterized in that** the actuating stroke, which is introduced into the housing top (37), continues via the scale ring, the planet-gear mechanism (12), and via the toothing formation (21), into the more or less flat-state indexing fingers (28) which, by engaging in the toothing formation (21) of the disk (15), rotate the latter, in the process springing over the stopping fingers (20), which prevent return rotation.

7. Step-action indexing mechanism according to one or more of the preceding claims, **characterized by** being disposed in a hand-held-unit inhaler such that the step-action indexing-mechanism housing (34), which is configured as a flat plate, including the hub (29) has a central hole (38) for the through-passage of the inhaler valve tube (5), and is latched on the hand-held-unit housing (2) such that it can still be displaced inward by the actuating stroke, the lower end surface of the hub (29) being supported in the process on the supporting portion (9), which has the central hole (38).

8. Hand-held appliance for apportioned delivery of inhaler medicaments through the outlet tubule of a cartridge which is displaceable for this purpose against opposed spring loading, **characterized by** a step-action indexing mechanism according to Claim 1 being arranged held on the cartridge and arranged in such a way that the spring loading of the cartridge outlet tubule is aligned for inherent spring loading of the step-action indexing fingers.

## Revendications

1. Mécanisme pas-à-pas, en particulier pour appareils portatifs (1) de délivrance fractionnée de médicaments à inhaler, ayant un boîtier annulaire autour de l'axe longitudinal (x-x) duquel tourne une bague graduée (27) en pas-à-pas sous l'action de doigts d'indexation, lesquels pivotent en direction d'un plan perpendiculaire à l'axe longitudinal (x-x) du boîtier (34) lors de la course d'actionnement à l'encontre d'une charge élastique et qui de ce fait provoquent par le biais d'une denture la rotation de la bague graduée, **caractérisé en ce qu'**une pièce annulaire (17) agencée bloquée en rotation dans le boîtier (34) présente au moins une fente (19) s'étendant à partir du bord inférieur en étant incliné vers le haut pour être engagée par un tenon de guidage (33) d'une étoile à doigts d'indexation pas-à-pas (S), les doigts d'indexation pas-à-pas (28) de cette dernière s'étendant de manière opposée et sécante à partir d'un moyeu central (29) en étant inclinée vers le haut et pénétrant par pivotement dans la denture (21) à l'encontre de leur tension élastique propre lors de la course d'actionnement pour faire tourner la bague graduée (26).

2. Mécanisme pas-à-pas selon la revendication 1, **caractérisé en ce que** la rotation de la bague graduée (26) est réalisée par un mécanisme planétaire (12) dont la roue satellite (13) est montée dans un alésage (24) de la bague d'indexation (26) et dont la roue planétaire intérieure (14) correspondante est disposée sur un disque (15) denté sur le dessous dont les dents sont engagées par les doigts d'indexation pas-à-pas (28), le mécanisme planétaire (12) transmettant l'angle de rotation du disque (15) en le démultipliant à la bague graduée (26).

3. Mécanisme pas-à-pas selon une ou plusieurs des revendications précédentes, **caractérisé en ce que** le moyeu (29) de l'étoile à doigts d'indexation pas-à-pas (S) présente un alésage central et est associé à une partie de support (9) avec sa surface frontale inférieure.

4. Mécanisme pas-à-pas selon une ou plusieurs des revendications précédentes, **caractérisé en ce que** les axes de tous les éléments d'indexation sont disposés perpendiculairement au plan de la bague graduée (26).

5. Mécanisme pas-à-pas selon une ou plusieurs des revendications précédentes, **caractérisé en ce que** le moyeu (30) est situé à distance du côté inférieur du fond du boîtier (34).

6. Mécanisme pas-à-pas selon une ou plusieurs des revendications précédentes, **caractérisé en ce que** la course d'actionnement appliquée au couvercle (37) du boîtier se transmet par le biais de la bague graduée, du mécanisme planétaire (12) et de la denture (21) jusqu'aux doigts d'indexation (28) qui s'étendent presque à plat, lesquels de par leur engagement dans la denture (21) du disque (15) font tourner le disque (15) par franchissement des dents par le doigt anti-retour (20).

7. Mécanisme pas-à-pas selon une ou plusieurs des revendications précédentes, **caractérisé par** l'agencement dans un inhalateur portatif de manière à ce que le boîtier (34) du mécanisme pas-à-pas réalisé sous forme d'assiette plate, y inclus le moyeu (29), présente un trou central (38) pour le passage du tube de soupape (5) de l'inhalateur et est fixé au boîtier (2) de l'inhalateur de manière à ce qu'il soit encore susceptible de se déplacer vers l'intérieur de la course d'actionnement sous appui de la surface inférieure du moyeu (29) sur la partie de support (9) laquelle présente le trou central (38).

8. Appareil portatif de délivrance fractionnée de médicaments à inhaler à travers un tube de délivrance d'une cartouche déplaçable à l'encontre d'une charge élastique, **caractérisé par** l'agencement d'un mécanisme pas-à-pas selon la revendication 1 lié à la cartouche et de manière à ce que la charge élastique du tube de délivrance de là cartouche soit alignée pour engendrer la tension élastique propre aux doigts d'indexation.
